# EUROPEAN PATENT APPLICATION

(11) **EP 1 316 288 A2**
(43) Date of publication of application: **04.06.2003**
(21) Application number: 02102556.4
(22) Date of filing: 11.11.2002
(51) Int. Cl.: A61B 5/0484

(54) **Device for presenting stimulus to test subjects for measuring brain function**

(30) Priority: 27.11.2001 JP 2001360455
(71) Applicant: Communications Research Laboratory, Independent Administrative Institution, Koganei-Shi, Tokyo 184-0015 (JP)
(72) Inventor: Eda, Hideo, 184-0015, Koganei-shi (JP); Tanabe, Hiroki, 184-0015, Koganei-shi (JP)
(74) Representative: Brunner, Michael John

(57) **Abstract**

A stimulus presentation device that provides stimuli to the test subject in a computer-based random manner during brain function measurement to investigate brain functions based on the response from a test subject exposed to stimuli. The invention can present stimuli to the test subject for measuring brain functions, eliminating time-dependent disturbance factors that may cause the test subject to become tired of the stimuli and accustomed to the repetition of stimuli during the test.

## Description

### FIELD OF THE INVENTION

The invention relates to a method of presenting stimulus to a test subject and the device thereof used to analyze brain functions based on reactions of the stimulated test subject.

### BACKGROUND OF THE INVENTION

Brain functions are occasionally tested by tasks that arouse the senses of sight, hearing, and touch in a test subject, then by observing the subsequent reactions. In such brain function tests, the brain's reaction to stimulus is measured in a noninvasive manner by, for example, using a functional magnet ic-resonance imaging (fMRI) system, magneto-encephalography(MEG), and optical imaging systems.

The MEG technique makes it possible to identify the distribution of magnetic-field response to stimulus and location of magnetic fields generated in the brain. In this way nerve activities can be measured noninvasively. Moreover, because this technique has a high time-resolution, brain activity can be monitored on the order of milliseconds. MEG information thereby indicates which part of the brain responds to a given stimulus. Meanwhile, fMRI is a technique used to measure the spin relaxation time of proton by applying a strong magnetic field to the brain. The relaxation time changes according to brain activity. This technique makes it possible to detect an increase in local blood flow and thereby indicate which part (texture) of the brain responds to a specific stimulus.

Such a stimulus is generally called a task. The test results significantly depend on how tasks are arranged. The state of a brain not subject to a task is called "rest" or "control." Based on the difference in brain conditions between a task and control, changes in the brain caused by a task are inferred and the brain functions understood.

While testing brain functions, it is necessary to check whether the tasks given to the test subject are causing target reactions. Because the brain's reaction to a task is very small, this check is made by accumulating the results of repeated tasks, and not by relying on the results of completing a single task. This accumulation is an important technique for making it easy to extract the target signal (which otherwise is hardly distinguishable from noise). Time-dependent factors should also be taken into account.

Tasks may be added up by, for example, repeating two tasks (task 1 and task 2, denoted T1 and T2) four times each for the same duration time along with rest (denoted R) in a task sequence such as Start -> R -> T1 -> T2 -> R -> T1 -> T2 -> R -> T1 -> T2 -> R -> T1 -> T2 -> End. However, it is known that such a sequence causes a systematic phenomenon by which the brain becomes accustomed to task 1 due to predictability, while the brain gets tired of task 2. Because it is very time-consuming to repeat several rounds of tasks, the accumulated fatigue resulting from lengthy tasks must be estimated. Moreover, when tasks are presented at fixed intervals, the fact that the brain becomes accustomed to a task pa ttern and the test subject will make a guess must also be considered.

The present invention is designed to solve such problems and provide a brain function measurement method that is not affected by such time-dependent factors as accumulated fatigue and an adaptation to repeated tasks. One aspect of the invention is therefore to provide a method of arousing a test subject for testing brain functions and effectively applying the stimulus presentation device thereof.

### SUMMARY OF THE INVENTION

The invention provides a stimulus presentation device for measuring brain functions. This device includes a monitor, speaker, or screen. This device comprises (for presenting multiple stimuli within a predetermined period) a means of randomly changing the sequence of stimuli presented using a computer, a means of randomly changing the intervals or duration of time for presenting said stimuli using a computer, and a means of controlling the stimulus presentation device so as to present the stimuli in said randomized sequence.

Another aspect of the invention is to provide a device to measure brain functions using functional magnetic -resonance imaging (fMRI) or near-infrared light. This device is connected with the stimulus presentation device of claim 1 and comprises a means of acquiring and recording stimulus-presenting information including the presented stimuli and presentation timing, as well as brain function measurement data on an accompanying computer. The stimuli to be presented to the test subject may include sounds, figures, and characters.

Other than to randomly present stimuli, the invention provides a device to measure brain functions consisting of a means of presenting stimulus (including a monitor, speaker or screen), a means of presenting a stimulus arousing the sense of sight for such a short time that the test subject cannot visually recognize said stimulus, a means of displaying multiple stimuli including said stimulus arousing the sense of sight so that the test subject can visually recognize the stimuli, a means of recording said stimulus information, and a means of acquiring and recording brain function information as a function of time during said stimulus presentation. Thus, the invention is capable of measuring brain functions to investigate sublimin al effects.

### BRIEF DESCRIPTION OF THE DRAWING

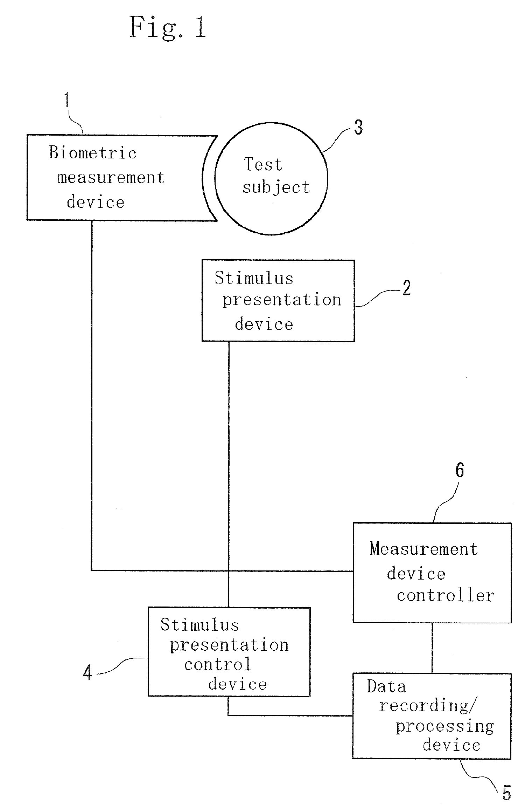

FIG.1 is a diagram showing the overall configuration of the measurement system of the invention; (1) denotes a biometric measurement device, (2) the stimulus presentation device, (3) the test subject, (4) the stimulus presentation control device, (5) the data recording/processing device, and (6) the measurement device controller.

### DETAILED DESCRIPTION OF THE PREFERRED EMBODIMENTS

The stimulus presentation device of the invention is a device that arouses the senses of, for example, sight and hearing of a test subject or test animal, while measuring brain activity by using the above-mentioned fMRI technique and near-infrared light. FIG.1 shows the overall configuration of the measurement device. This device monitors brain activity in a noninvasive manner by fixing test subject (3) in a predetermined position and using a biometric measurement device (1) (e.g., one using fMRI, MEG, near-infrared light). Measurement device controller (6) controls the measurement device. Stimulus presentation device (2) (e.g., monitor, screen, speaker) provides stimuli to the test subject. Stimulus presentation control device (4) controls how to present stimuli (randomly or otherwise) as desired. The measured biometric data and stimulus presenting conditions are recorded and processed as a function of time by data recording/processing device (5).

When a human attempts to provide stimuli, the test subject can predict or guess the stimulating pattern. Consequently, reproducible and objective data is not created from the stimuli given by a task. Such problems can be solved by the invention.

When arousing the sense of sight in measuring brain activity, we may choose figures and characters for display on a monitor, and may change the duration of presentation. For example, it is possible to change the intensity of stimuli by increasing or decreasing the frequency of alternatively displaying a checkerboard and a blank image on the screen at predetermined intervals. When a human tries to manually change the frequency or arrangement of tasks, the changed pattern is likely to be fixed and the procedures rather complex.

Thus the invention uses random numbers generated by a computer to randomize the selection of figures and other information displayed on the monitor, as well as the stimulating intensity. Such figures may be characters and motion pictures, and may be presented together with sound.

The figures and stimulating intensity selected are recorded together with the lapse of time in computer memory. Such information may be recorded in relation to brain function measurement results.

The sense of sight can be stimulated using an ordinary monitor. Otherwise, a monitor installed inside goggles may be used to arouse the sense of sight over a wide range. The use of goggles makes it possible to selectively stimulate either eye. Moreover, it is also possible to prepare a box with two sight holes for the eyes and let the test subject look at images displayed in the box. In this way the sense of sight can be stimulated over a wide range, and either eye can be selectively exposed to stimuli.

When arousing the sense of hearing, we can use a speaker or similar device to provide stimuli by randomly changing the different sounds, volume, and duration of the stimulus being presented.

In the above configuration, the computer generates random numbers on which the stimuli are based. Thus the test can be conducted objectively. Stimuli are thereby presented to the brain on a random basis during brain function mea surement.

Next explained is a device that measures brain functions by providing patterned stimuli. In contrast to the above random presentation, by presenting stimuli in a patterned procedure, it is possible to see how humans become accustomed to stimuli and make a guess.

For example, we can study how brain activity changes as the test subject becomes accustomed to stimuli when exposed to a fixed figure presented at constant intervals. It is also possible to study the brain's latent capabilities by presenting figures to a test subject at high speed (for a very short duration) so that the test subject can visually recognize those figures, even unconsciously, then checking whether the brain responds to any of the figures presented.

Moreover, the subliminal effects of recent interest can be examined by brain measurement. Specifically, a predetermined figure embedded in the frame of a video movie is presented to a test subject, and after the movie ends, some figures are presented to the test subject for a period of time until the test subject manages to visually recognize them. By measuring brain activity while presenting such a movie to a test subject, we can analyze how the brain works and identify the active areas of the brain.

It has been difficult to provide stimuli to the brain in a perfectly random manner, and such experiments have required a complex arrangement of tasks. In the present invention, however, a computer generates random numbers and provides stimuli to the test subject based on the random numbers generated. Consequently, such problems as the test subject becoming accustomed to a given stimuli and guessing at what kind of stimulus comes next can be solved, and objective, reproducible test results obtained. Moreover, because the computer stores and processes the randomized stimulating patterns and corresponding changes in brain activity, the test results can be easily analyzed.

Moreover, the manner of presenting stimuli is not limited to random presentation. For example, if a specific figure is instantaneously presented for such a short time that the test subject cannot visually recognize it, the brain's response to the visually unrecognizable figure can be investigated.

## Claims

1. A stimulus presentation device for enabling presentation of multiple stimuli within a predetermined period, the device including
a means of randomly changing the sequence of stimuli presented using a computer;
a means of randomly changing the intervals or durations of time for presenting said stimuli using a computer; and
a means of controlling the stimulus presentation device so as to present the stimuli in said randomized sequence.

2. A device to measure brain functions using functional magnetic resonance imaging (fMRI) or near-infrared light and including a device according to claim 1, and further comprising a means of acquiring and recording stimulus-presenting information including the presented stimuli and presentation timing, as well as brain function measurement data, on an accompanying computer.

3. A device to measure brain functions comprising:
a means of presenting a stimulus to arouse the sense of sight for such a short time that the test subject cannot visually recognize said stimulus;
a means of displaying multiple stimuli including said stimulus arousing the sense of sight so that the test subject can visually recognize the stimuli;
a means of recording said stimulus information; and
a means of acquiring and recording brain function information as a function of time during said stimulus presentation.

4. A device according to claim 1, further comprising an output device including one or more of a monitor, a speaker or a screen.

5. A device according to claim 3, wherein the means of presenting a stimulus includes a monitor or screen.
